# EUROPEAN PATENT SPECIFICATION

(11) **EP 2 533 043 B1**
(45) Date of publication and mention of the grant of the patent: **02.11.2022**
(21) Application number: 11739768.7
(22) Date of filing: 02.02.2011
(51) Int. Cl.: G01N 30/88, G01N 30/34, G01N 30/96

(54) **HEMOGLOBIN S ANALYSIS METHOD**
HÄMOGLOBIN-S-ANALYSEVERFAHREN
PROCÉDÉ D'ANALYSE D'HÉMOGLOBINE S

(30) Priority: 02.12.2010 JP 2010269551; 02.02.2010 JP 2010021466
(43) Date of publication of application: 12.12.2012
(73) Proprietor: Sekisui Medical Co., Ltd., Tokyo 103-0027 (JP)
(72) Inventor: TAIRA Hiroaki, Ryugasaki-shi Ibaraki 301-0852 (JP); OKA Takayuki, Ryugasaki-shi Ibaraki 301-0852 (JP)
(74) Representative: Cabinet Beau de Loménie
(86) International application number: PCT/JP2011/052113
(87) International publication number: WO 2011/096420

(56) References cited:
- EP-A1- 1 103 812
- JP-A- 2 236 164
- JP-A- 2003 014 714
- JP-A- 2003 107 069
- JP-A- 2007 078 361
- JP-A- 2007 315 816
- US-A- 4 980 058
- R.S.ERSSER ET AL.: 'Automated Quantitative Microcolumn Chromatography of Haemoglobin A2' BIOMEDICAL CHROMATOGRAPHY vol. 5, no. 5, September 1991, pages 226 - 228, XP026539626
- MARIA BEATRIZ DE LA CALLE GUNTINAS ET AL.: 'Determination of haemoglobin Alc by liquid chromatography using a new cation-exchange column' JOURNAL OF CHROMATOGRAPHY B vol. 791, no. 1-2, 05 July 2003, pages 73 - 83, XP004428032

## Description

### TECHNICAL FIELD

The present invention relates to a hemoglobin S analysis method which enables even highly retentive hemoglobin S to be separated in sharp, highly symmetrical peaks by cation-exchange high-performance liquid chromatography.

### BACKGROUND ART

High-performance liquid chromatography (HPLC) analysis of hemoglobins is a widely used technique. Specifically, this technique is used for diagnosis of diabetes, for example, to quantify a glycohemoglobin, hemoglobin A1c, or to analyze abnormal hemoglobins like according to EP 1 103 812 A1 with an eluent buffer containing sodium azide and in a pH range from 4.0 to 6.8 and according to JP 2003-014714 A or JP 2003-107069 A with eluent buffers having azide concentration ranges from 0.15 to 6.15 mmol/l or 50 mmol/l, respectively. For example, JP 2000-111539 A discloses a method utilizing liquid chromatography which separates hemoglobin components in a diluted hemolyzed blood sample by a cation-exchange method based on the difference in positive charge between the hemoglobin components. A recent increase in diabetes patients has also increased the number of cases requiring hemoglobin A1c analysis. This tendency has created a demand for more accurate, less time-consuming HPLC analysis.

Hemoglobins are present in the body in the forms of oxyhemoglobin that contains bound oxygen, deoxyhemoglobin that contains bound carbon dioxide, and methemoglobin in which the iron in the heme group is oxidized into the trivalent ion state. It is known that in the presence of an azide or cyanide, the trivalent Fe ion in methemoglobin binds to the azide or cyanide, resulting in the conversion of methemoglobin into stable azide metohemoglobin or cyanomethemoglobin. Disadvantageously, in the case of cation-exchange HPLC, oxyhemoglobin may differ from azide metohemoglobin or cyanomethemoglobin in elution time. Because of a slight difference in electric charge between these hemoglobin forms, the HPLC analysis may result in poorly separated broad peaks or a bimodal distribution.

HPLC analysis of hemoglobins is mainly used for diagnosis of hemoglobinopathy and thalassemia which may cause anemia, in addition to diabetes. Especially, the number of cases requiring analysis and detection of hemoglobin S is large because hemoglobin S is the most common abnormal hemoglobin and causes sickle cell anaemia which results in severe anemia. On the other hand, in the case of analysis of the diabetes marker hemoglobin A1c, it is preferred to separate abnormal hemoglobins including hemoglobin S. If the analysis provides broad peaks or a bimodal distribution, separation of these abnormal hemoglobins from normal hemoglobins is difficult and these hemoglobins may have a negative impact on the resulting measurements. Therefore, it is preferred to separate these abnormal hemoglobins in sharp peaks. In the case of diagnosis of thalassemia, hemoglobin A2 is analyzed. Hemoglobin A2 is, however, a minor component and often elutes next to hemoglobin A0 that is present in a large amount. Thus, it is preferred to separate both hemoglobin A0 and hemoglobin A2 in sharp peaks. However, in the case of cation-exchange chromatography, components that are comparatively retentive in a cation-exchange column may cause the problem of broad peaks or a bimodal distribution.

Further, deteriorated blood samples tend to give broad peaks or a bimodal peak distribution compared to fresh blood samples. This is because the amount of metohemoglobin is increased due to deterioration. Therefore, in the case of analysis of a preserved sample (e.g. re-examination), there is a possibility of a negative impact on the resulting measurements.

### SUMMARY OF INVENTION

### - Technical Problem

The object of the present invention is to provide a hemoglobin S analysis method according to claim 1 and the claims depending thereon, which enables even highly retentive hemoglobin S to be separated in sharp, highly symmetrical peaks by cation-exchange high-performance liquid chromatography.

### - Solution to Problem

A first aspect, which is the aspect of the present invention, is a method of claim 1 for analyzing hemoglobin S by cation-exchange high-performance liquid chromatography, which includes utilizing an eluent that contains an azide or a cyanide at a concentration of 0.1 to 50 mmol/L and has a pH in the range of 6.80 to 7.50 near the isoelectric point of hemoglobin.

A second aspect not of the present invention is a method for analyzing hemoglobin A2 by cation-exchange high-performance liquid chromatography, which includes utilizing an eluent that contains an azide or a cyanide at a concentration of 0.1 to 50 mmol/L and has a pH in the range of 6.45 to 6.85 near the isoelectric point of hemoglobin.

A third aspect not of the present invention is a method for analyzing hemoglobin A0 by cation-exchange high-performance liquid chromatography, which includes utilizing an eluent that contains an azide or a cyanide at a concentration of 0.1 to 50 mmol/L and has a pH in the range of 6.00 to 6.75 near the isoelectric point of hemoglobin.

The following description discusses the above-mentioned aspects in detail.

Generally, eluents having a pH of less than 6 have been used to separate highly retentive hemoglobins, whereas the above pH range has a large impact on the shape of peaks, and even highly retentive hemoglobins can be separated in highly symmetry sharp peaks by using an eluent that contains an azide or cyanide at a specific concentration to stabilize methemoglobin and is adjusted to a pH in a certain range near the isoelectric point of hemoglobin.

The term "highly retentive hemoglobins" herein is intended to mean hemoglobin A0, hemoglobin A2, and hemoglobin S which exhibit high retention in a cation-exchange column. It is known that the isoelectric points of hemoglobin A0, hemoglobin A2, and hemoglobin S are in the range of 6.95 to 7.45. The term "poorly retentive hemoglobins" is intended to mean hemoglobins which exhibit low retention in a cationic-exchange column, and specifically refer to hemoglobin A1a, hemoglobin A1b, hemoglobin F, labile hemoglobin A1c, stable hemoglobin A1c, and the like. It should be noted that the order of elution of hemoglobins in ion-exchange chromatography does not always correspond to their isoelectric points because the retention of hemoglobins depends on their three dimensional structure.

In the hemoglobin S analysis method of the first aspect of the present invention, the hemoglobin A2 analysis method of the second aspect, and the hemoglobin A0 analysis method of the third aspect, an eluent containing an azide or a cyanide is used.

Since the eluent contains an azide or cyanide, methemoglobin is stabilized. Generally, hemoglobins are quantified based on their absorbance of a wavelength near 415 nm. The difference in absorption spectra at a wavelength near 415 nm of oxyhemoglobin and azide hemoglobin or cyanomethemoglobin is too small to be a problem in the accuracy of quantification. On the other hand, if the eluent does not contain azides and cyanides, hemoglobins are present in the methemoglobin form, which is known to have a considerably prolonged elution time in cation-exchange high-performance liquid chromatography. In addition, methemoglobin may cause a problem in the accuracy of quantification at 415 nm because the local maximum of the absorbance, although depending on the external environment, is near 405 nm.

Examples of the azide include sodium azide, diphenylphosphoryl azide, 4-dodecylbenzenesulfonyl azide, 4-acetylamidobenzenesulfonyl azide, potassium azide, lithium azide, iron azide, hydrogen azide, lead azide, mercury azide, copper azide, and silver azide.

Examples of the cyanide include potassium cyanide, hydrogen cyanide, sodium cyanide, silver cyanide, mercury cyanide, copper cyanide, lead cyanide, iron cyanide, lithium cyanide, and ammonium cyanide.

In the hemoglobin S analysis method of the present invention, the lower limit of the azide or cyanide concentration in the eluent is 0.1 mmol/L, and the upper limit thereof is 50 mmol/L. If the azide or cyanide concentration is lower than 0.1 mmol/L, the methemoglobin stabilization effect is not enough. If the azide or cyanide concentration is higher than 50 mmol/L, excessive met-form transformation and/or decomposition of hemoglobins may arise. The preferable lower limit of the azide or cyanide concentration is 0.5 mmol/L, and the preferable upper limit is 30 mmol/L. The more preferable lower limit is 1 mmol/L, and the more preferable upper limit is 10 mmol/L.

The use of the hemoglobin S analysis method of the first aspect of the present invention enables even highly retentive hemoglobin S to be separated in a sharp, highly symmetrical peak.

In the hemoglobin S analysis method of the present invention, the upper limit thereof is 7.50. If the pH of the eluent is less than 6.80, hemoglobin S analysis by HPLC may result in a broad leading peak, a broad peak, or a bimodal distribution. If the pH of the eluent is more than 7.50, hemoglobin S may exhibit low retention in a cation-exchange column and thus may be eluted in an extremely short time, or the analysis may result in a broad tailing peak, a broad peak, or a bimodal distribution. In the hemoglobin S analysis method of the present invention, the preferable lower limit of the pH of the eluent is 6.95, and the preferable upper limit is 7.45. The more preferable lower limit is 7.00, and the more preferable upper limit is 7.40.

In the hemoglobin S analysis method of the present invention, the eluent is not particularly limited, provided that the azide or cyanide concentration and the pH fall within the above-mentioned respective ranges. The eluent may be, for example, a known buffer containing a buffering agent such as an organic acid or a salt thereof, an amino acid, an inorganic acid or a salt thereof, or a Good's buffer.

Examples of the organic acid include citric acid, succinic acid, tartaric acid, and malic acid.

Examples of the amino acid include glycine, taurine, and arginine.

Examples of the inorganic acid include hydrochloric acid, nitric acid, sulfuric acid, phosphoric acid, boric acid, and acetic acid.

The buffer may optionally contain any of surfactants, various polymers, hydrophilic low-molecular weight compounds, and the like.

In the hemoglobin S analysis method of the present invention, the buffering agent concentration in the eluent is not particularly limited, but the preferable lower limit thereof is 5 mmol/L, and the preferable upper limit thereof is 500 mmol/L. If the buffering agent concentration is lower than 5 mmol/L, the buffer action may not be enough. If the buffering agent concentration is higher than 500 mmol/L, the buffering agent may be precipitated so as to clog an HPLC path and reduce the eluent replacement efficiency, resulting in a longer time for equilibration. The more preferable lower limit of the buffering agent concentration is 10 mmol/L, and the preferable upper limit is 200 mmol/L.

In order to optimize elution of hemoglobins in peaks, the eluent may contain an inorganic salt such as sodium perchlorate, sodium chloride, potassium chloride, sodium sulfate, potassium sulfate, sodium phosphate, or sodium thiocyanate.

In the hemoglobin S analysis method of the present invention, the salt concentration in the eluent is not particularly limited, but the preferable upper limit thereof is 500 mmol/L. If the salt concentration is higher than 500 mmol/L, the salt may be precipitated to cause a negative impact on an analysis system. The more preferable upper limit of the salt concentration is 200 mmol/L.

The eluent may contain a pH adjuster such as a known acid or base. Examples of the acid include hydrochloric acid, phosphoric acid, nitric acid, and sulfuric acid. Examples of the base include sodium hydroxide, potassium hydroxide, lithium hydroxide, magnesium hydroxide, barium hydroxide, and calcium hydroxide.

The eluent may contain a water-soluble organic solvent such as methanol, ethanol, acetonitrile, or acetone. The water-soluble organic solvent is preferably added at a concentration that does not cause components such as the salt to be precipitated, and the preferable upper limit of the concentration is 80% (v/v).

Highly retentive hemoglobin S is eluted with the above eluent by the hemoglobin S analysis method of the present invention. Before elution with this eluent, poorly retentive hemoglobins may be eluted with an eluent having a pH less than this eluent. In this case, eluents to be used are preferably buffers that contain the same components, but are not limited only to buffers that contain the same components, provided that baseline variations of detector outputs caused by eluent changes have no impact on the resulting measurements.

More preferably, the eluents have the same buffering agent concentration in order to further reduce the baseline variations.

In the hemoglobin S analysis method of the present invention, cation-exchange high-performance liquid chromatography is employed. The cation-exchange high-performance liquid chromatography may be performed in a known manner, for example, by conveying the eluent to a cation-exchange column through a degasser by a pump to separate hemoglobins maintained in the cation-exchange column, and analyzing a mobile phase flowing out of the cation-exchange column.

The cation-exchange column used in the hemoglobin S analysis method of the present invention is a column containing a fixed phase. Examples of the fixed phase include filler particles and porous materials, and filler particles are preferred.

Examples of the filler particles include inorganic particles and organic particles.

Examples of the inorganic particles include particles made of silica, zirconia, or the like.

Examples of the organic particles include natural polymer particles of cellulose, a polyamino acid, chitosan, or the like, and synthetic polymer particles of polystyrene, a polyacrylic acid ester, or the like.

The fixed phase is preferably a fixed phase that has a cation-exchange group.

Examples of the cation-exchange group include carboxyl group, phosphate group, and sulfone group.

The analysis conditions of the hemoglobin S analysis method of the present invention can be appropriately determined based on samples to be analyzed, the type of the cation-exchange column, and the like. Specifically, the preferable lower limit of the flow rate of the eluent is 0.05 mL/min, and the preferable upper limit thereof is 5 mL/min. The more preferable lower limit is 0.2 mL/min, and the more preferable upper limit is 3 mL/min. The detection wavelength for hemoglobins is preferably, but is not limited only to, 415 nm. Generally, samples to be analyzed are those prepared by hemolyzing a blood sample with a solution that contains a substance having a hemolytic activity such as a surfactant, and diluting the hemolyzed sample. The amount of a sample to be introduced depends on the dilution ratio of the blood sample and is preferably about 0.1 to 100 µL.

### - Advantageous Effects of Invention

The present invention provides a hemoglobin S analysis method which enables even highly retentive hemoglobin S to be separated in sharp, highly symmetrical peaks by cation-exchange high-performance liquid chromatography.

### BRIEF DESCRIPTION OF DRAWINGS

Fig. 1 is a graph illustrating the relationship between the pH and the sodium perchlorate concentration of eluents which were adjusted to give a hemoglobin S elution time of 50 seconds.
Figs. 2(a), 2(b), and 2(c) are respectively chromatograms of sample A, sample B, and sample C each of which was eluted using eluent 2 for a period of time ranging from 0.5 minutes to 1.0 minute after starting analysis.
Figs. 3(a), 3(b), and 3(c) are respectively chromatograms of sample A, sample B, and sample C each of which was eluted using eluent 3 for a period of time ranging from 0.5 minutes to 1.0 minute after starting analysis.
Figs. 4(a), 4(b), and 4(c) are respectively chromatograms of sample A, sample B, and sample C each of which was eluted using eluent 4 for a period of time ranging from 0.5 minutes to 1.0 minute after starting analysis.
Figs. 5(a), 5(b), and 5(c) are respectively chromatograms of sample A, sample B, and sample C each of which was eluted using eluent 5 for a period of time ranging from 0.5 minutes to 1.0 minute after starting analysis.
Figs. 6(a), 6(b), and 6(c) are respectively chromatograms of sample A, sample B, and sample C each of which was eluted using eluent 6 for a period of time ranging from 0.5 minutes to 1.0 minute after starting analysis.
Figs. 7(a), 7(b), and 7(c) are respectively chromatograms of sample A, sample B, and sample C each of which was eluted using eluent 7 for a period of time ranging from 0.5 minutes to 1.0 minute after starting analysis.
Figs. 8(a), 8(b), and 8(c) are respectively chromatograms of sample A, sample B, and sample C each of which was eluted using eluent 8 for a period of time ranging from 0.5 minutes to 1.0 minute after starting analysis.
Figs. 9(a), 9(b), and 9(c) are respectively chromatograms of sample A, sample B, and sample C each of which was eluted using eluent 9 for a period of time ranging from 0.5 minutes to 1.0 minute after starting analysis.
Figs. 10(a), 10(b), and 10(c) are respectively chromatograms of sample A, sample B, and sample C each of which was eluted using eluent 10 for a period of time ranging from 0.5 minutes to 1.0 minute after starting analysis.
Figs. 11(a), 11(b), and 11(c) are respectively chromatograms of sample A, sample B, and sample C each of which was eluted using eluent 11 for a period of time ranging from 0.5 minutes to 1.0 minute after starting analysis.
Figs. 12(a), 12(b), and 12(c) are respectively chromatograms of sample A, sample B, and sample C each of which was eluted using eluent 12 for a period of time ranging from 0.5 minutes to 1.0 minute after starting analysis.
Figs. 13(a), 13(b), and 13(c) are respectively chromatograms of sample A, sample B, and sample C each of which was eluted using eluent 13 for a period of time ranging from 0.5 minutes to 1.0 minute after starting analysis.
Fig. 14 is a graph illustrating the relationship between the pH of eluents 2 to 13 and the symmetry coefficient of peaks 2 in the analyses of sample A.
Fig. 15 is a graph illustrating the relationship between the pH of eluents 2 to 13 and the difference in elution time between peaks 2 corresponding to sample A and peaks 3 corresponding to sample B.
Fig. 16 is a graph illustrating the relationship between the pH of eluents 2 to 13 and the resolution of peaks 2 in the analyses of sample A.
Fig. 17 is a graph illustrating the relationship between the pH of eluents 2 to 13 and the depth of valleys between peaks 1 and peaks 2 in the analyses of sample A.

### DESCRIPTION OF EMBODIMENTS

The following description will discuss the present invention in more detail by way of Examples, but the scope of the present invention is not limited only to these examples.

### (Example 1)

The following three samples were analyzed.

Sample A was prepared by diluting a blood sample containing hemoglobin S 100-fold with a diluent (phosphate buffer (pH 7.00) containing 0.1% Triton X-100).

Sample B was prepared by diluting AFSC control (Helena Laboratories) 50-fold with a diluent (phosphate buffer (pH 7.00) containing 0.1% Triton X-100).

Sample C was prepared by mixing sample A and sample B at 1:1.

The used cation-exchange column was one containing a cation-exchange resin, and the used HPLC instrument was provided with a detector SPD-M20A (Shimadzu Corp.), a sample delivery pump LC-20AD (Shimadzu Corp.), a degasser DGU-20A5 (Shimadzu Corp.), a column oven CTO-20AC (Shimadzu Corp.), and an autosampler SIL-20AC (Shimadzu Corp.). The analysis was performed under the following conditions:
flow rate: 1.7 mL/min;
detection wavelength: 415 nm; and
amount of introduced sample: 10 µL.

Each sample was eluted using the following eluents for the respective periods of time:
from 0 (start) to 0.5 minutes after the start: eluent 1 (40 mmol/L phosphate buffer (pH 5.35) containing 60 mmol/L sodium perchlorate and 1 mmol/L sodium azide);
from 0.5 minutes to 1.0 minute after the start: eluent 2 shown in Table 1;
from 1.0 minute to 1.1 minutes after the start: eluent 14 (40 mmol/L phosphate buffer (pH 8.00) containing 0.8% by weight of Triton X-100, 30 mmol/L sodium perchlorate, and 1 mmol/L sodium azide); and
from 1.1 minutes to 1.5 minutes after the start: eluent 1.

The buffering agent concentration in eluent 2 was controlled such that the sample A analysis resulted in a hemoglobin S elution time of about 50 seconds.

The detection was at 415 nm.

### (Examples 2 to 5 and Comparative Examples 1 to 7)

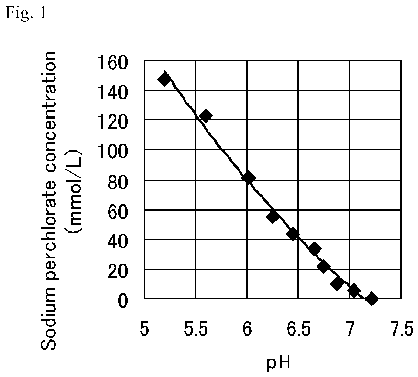

Samples A, B, and C were analyzed in the same manner as in Example 1, except that eluents 3 to 13 shown in Table 1 were used for elution over the period of time ranging from 0.5 minutes to 1.0 minute after the start. The buffering agent concentration in eluent 3 was controlled such that the sample A analysis resulted in a hemoglobin S elution time of about 50 seconds. The pH and salt concentration in eluents 4 to 13 were controlled such that the sample A analyses resulted in a hemoglobin S elution time of about 50 seconds (Fig. 1).

### <Evaluation>

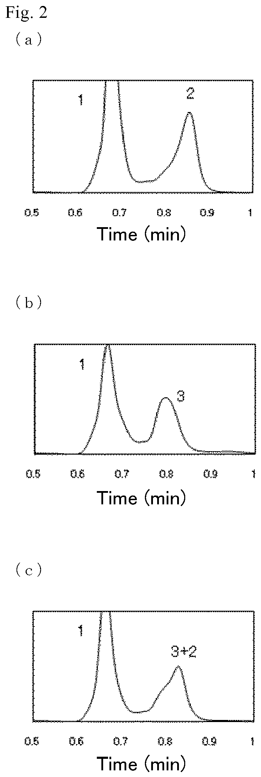

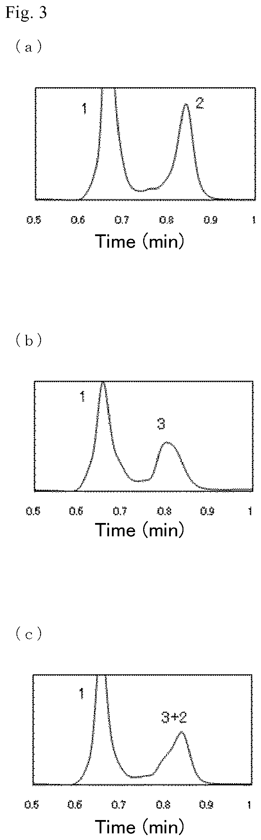

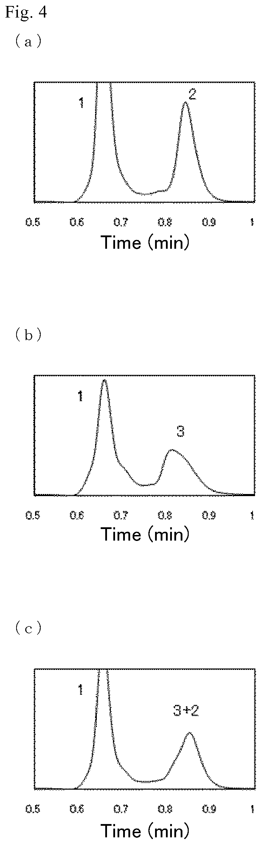

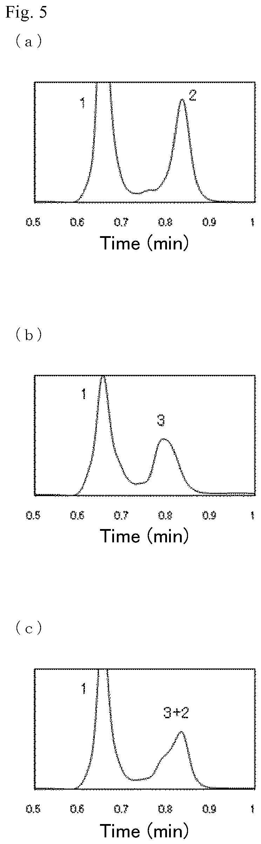

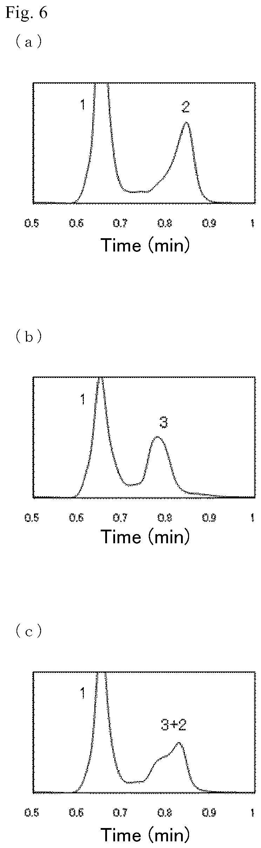

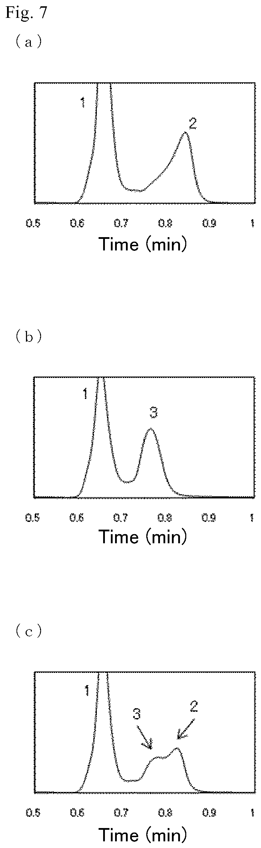

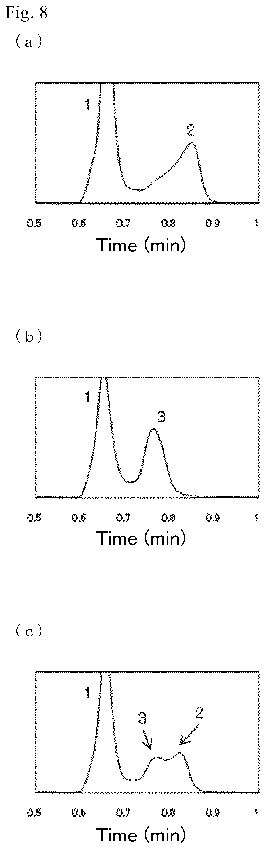

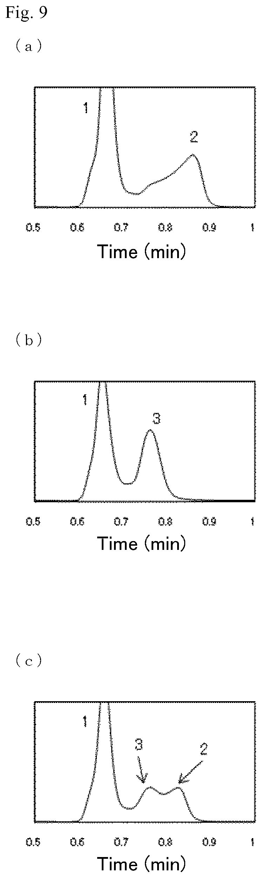

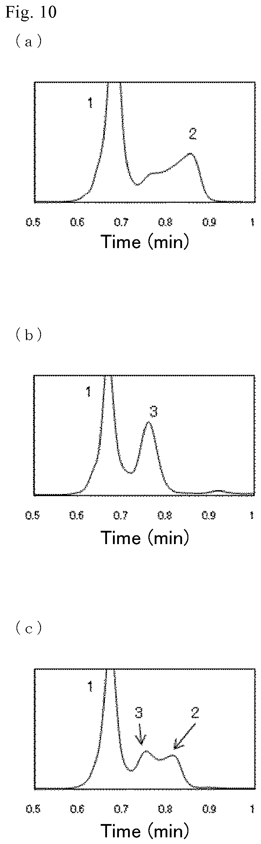

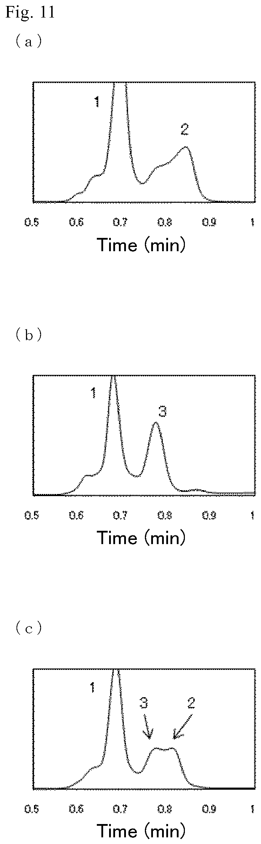

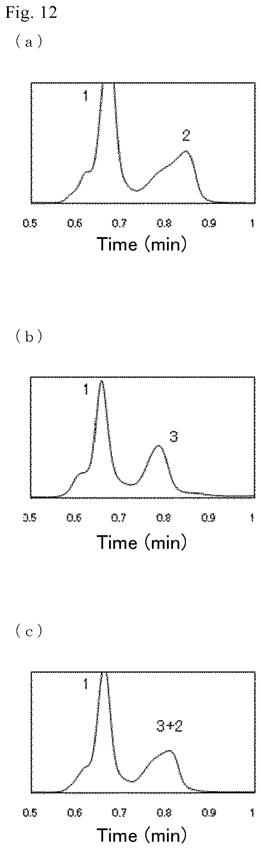

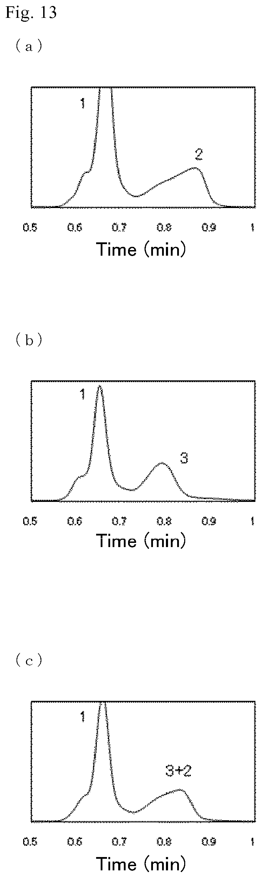

Figs. 2 to 13 are partial chromatograms of samples A, B, and C covering the period of time ranging from 0.5 minutes to 1.0 minute after the start in which eluents 2 to 13 were delivered in Examples 1 to 5 and Comparative Examples 1 to 7. In Figs. 2 to 13, peaks 1 correspond to hemoglobin A0, peaks 2 correspond to hemoglobin S in the oxy form, and peaks 3 correspond to azide methemoglobin S. It should be noted that common hemoglobin S-containing samples result in chromatograms similar to those of sample A, which means that they are rich in hemoglobin in the oxy form. In sample B, most hemoglobin is transformed into the met form, that is, sample B is rich in methemoglobin. This sample is in a state similar to that of a remarkably deteriorated normal sample. Sample C contains hemoglobin in the oxy form and methemoglobin at similar levels. This sample was used to test a condition that tends to give a bimodal distribution of peaks.

Figs. 2 to 13 demonstrate that the use of eluents 2 to 6 gave chromatograms in each of which peak 2 corresponding to sample A is sharp, and also demonstrate that sample C gave bimodal distributions of peaks 2 and 3 when eluents 7 to 11 were used.

### (Peak shape)

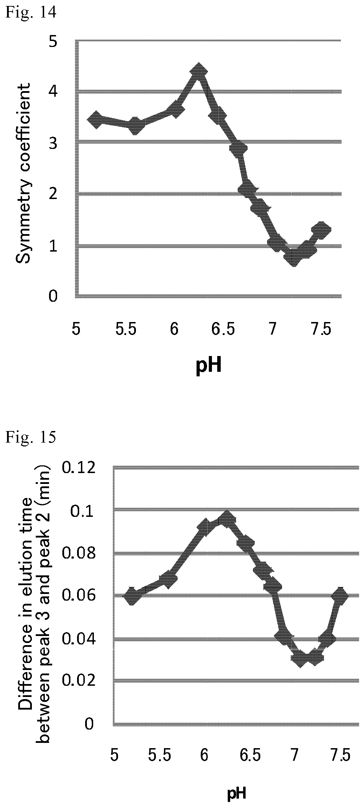

A symmetry coefficient was calculated for peaks 2 of sample A. A symmetry coefficient closer to 1 indicates a peak shape closer to a normal distribution; thus the symmetry coefficient was used as an indicator of peak shape. Generally, the peak width at a height of 5% of the peak height is used to calculate the symmetry coefficient. However, in these examples, the coefficient was calculated using the half-width value because peaks 1 upstream of peaks 2 are fused with peaks 2 and the peak width at 5% height could not be calculated. The results are presented in Table 2. Fig. 14 is a graph illustrating the relationship between the pH of eluents 2 to 13 and the symmetry coefficient of peaks 2 in the analyses of sample A. Fig. 14 demonstrates that a pH closer to 7 near the isoelectric point of hemoglobin corresponds to a symmetry coefficient closer to 1, and therefore indicates a highly symmetrical peak.

In addition, the difference in elution time between peaks 2 of sample A and peaks 3 of sample B was calculated. The difference in elution time between peaks 2 of sample A and peaks 3 of sample B corresponds to the difference in elution time between hemoglobin S in the oxy form and azide methemoglobin. A smaller difference corresponds to elution times similar to each other, and therefore indicates peaks combined into a single peak; thus the difference was used as an indicator of the peak shape in combination with the symmetry coefficient. The results are presented in Table 2. Fig. 15 shows the relationship between the pH of eluents 2 to 13 and the difference in elution time between peaks 2 of sample A and peaks 3 of sample B which was calculated based on the analysis results of samples A and B. Fig. 15 demonstrates that the elution times of peak 2 and peak 3 are most close to each other approximately at pH 7.

### (Resolution between adjacent peaks)

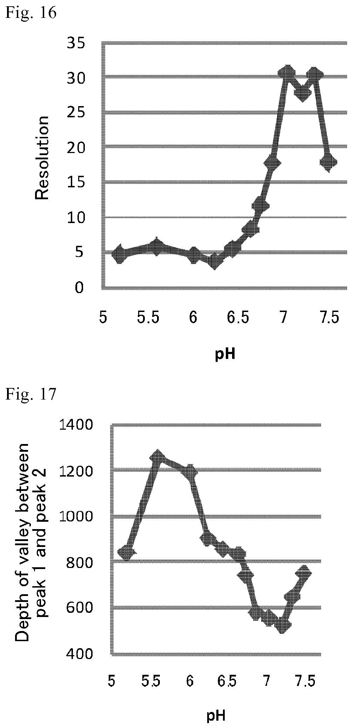

The resolution was calculated for peaks 2 of sample A by the JP (Japanese Pharmacopoeia) method. The results are presented in Table 2. Fig. 16 shows the relationship between the pH of eluents 2 to 13 and the resolution of peaks 2 in the analyses of sample A. Fig. 16 demonstrates that the resolution is higher at a pH closer to 7 near the isoelectric point of hemoglobin, and namely demonstrates that peak 1 and peak 2 are resolved well at such a pH.

In addition, the depth of the valleys between peaks 1 and peaks 2 obtained in the analyses of sample A was calculated. The depth of the valleys between peaks 1 and peaks 2 in the analyses of sample A was used as an indicator for the resolution between adjacent peaks in combination with the resolution. The depth of each of the valleys between peaks 1 and peaks 2 was determined as the lowest point between each pair of peaks 1 and 2. The results are presented in Table 2. Fig. 17 shows the relationship between the pH of eluents 2 to 13 and the depth of the valleys between peaks 1 and peaks 2 in the analyses of sample A. Fig. 17 demonstrates that the depth is deeper at a pH closer to 7, and namely indicates that peaks 1 and peak 2 are resolved well at such a pH.

**[Table 1]**

| | Eluent | Phosphate buffer concentration (mmol/L) | Sodium perchlorate concentration (mmol/L) | Sodium azide concentration (mmol/L) | pH |
|---|---|---|---|---|---|
| Example 1 | Eluent 2 | 5 | 0 | 1 | 7. 50 |
| Example 2 | Eluent 3 | 15 | 0 | 1 | 7. 35 |
| Example 3 | Eluent 4 | 25 | 0 | 1 | 7. 22 |
| Example 4 | Eluent 5 | 25 | 6 | 1 | 7. 05 |
| Example 5 | Eluent 6 | 25 | 10 | 1 | 6. 88 |
| Comparative Example 1 | Eluent 7 | 25 | 22 | 1 | 6. 75 |
| Comparative Example 2 | Eluent 8 | 25 | 33 | 1 | 6. 65 |
| Comparative Example 3 | Eluent 9 | 25 | 44 | 1 | 6. 45 |
| Comparative Example 4 | Eluent 10 | 25 | 55 | 1 | 6. 25 |
| Comparative Example 5 | Eluent 11 | 25 | 81 | 1 | 6. 02 |
| Comparative Example 6 | Eluent 12 | 25 | 125 | 1 | 5. 60 |
| Comparative Example 7 | Eluent 13 | 25 | 147 | 1 | 5. 20 |

**[Table 2]**

| | Eluent | pH | Resolution pattern of peak 2 (Peak shape) (Sample A) | Symmetry coefficient of peak 2 (Sample A) | Resolution of Peak 2 (Sample A) | Depth of valley between peak 1 and peak 2 (Sample A) | Difference in elution time between peak 2 (sample A) and peak 3 (sample B) (min) |
|---|---|---|---|---|---|---|---|
| Example 1 | Eluent 2 | 7.50 | Well resolved | 1.81 | 17.83 | 752 | 0.060 |
| Example 2 | Eluent 3 | 7.35 | Well resolved | 1.30 | 30.29 | 648 | 0.040 |
| Example 3 | Eluent 4 | 7.22 | Well resolved | 0.80 | 27. 75 | 526 | 0.031 |
| Example 4 | Eluent 5 | 7.05 | Well resolved | 1.06 | 30.57 | 553 | 0.031 |
| Example 5 | Eluent 6 | 6.88 | Well resolved | 1.72 | 17.69 | 579 | 0.041 |
| Comparative Example 1 | Eluent 7 | 6.75 | Leading | 2.10 | 11.58 | 745 | 0.064 |
| Comparative Example 2 | Eluent 8 | 6.65 | Leading | 2.91 | 8.14 | 836 | 0.072 |
| Comparative Example 3 | Eluent 9 | 6.45 | Bimodal distribution | 3.54 | 5.44 | 858 | 0.085 |
| Comparative Example 4 | Eluent 10 | 6.25 | Bimodal distribution | 4.41 | 3.68 | 909 | 0.096 |
| Comparative Example 5 | Eluent 11 | 6.02 | Bimodal distribution | 3.67 | 4.46 | 1196 | 0.093 |
| Comparative Example 6 | Eluent 12 | 5.60 | Bimodal distribution | 3.36 | 5.78 | 1259 | 0.068 |
| Comparative Example 7 | Eluent 13 | 5.20 | Leading | 3.46 | 4.63 | 844 | 0.060 |

### INDUSTRIAL APPLICABILITY

The present invention provides a hemoglobin S analysis method which enables even highly retentive hemoglobin S to be separated by cation-exchange high-performance liquid chromatography.

### REFERENCE SIGNS LIST

- 1: Hemoglobin A0
- 2: Hemoglobin S in oxy form
- 3: Azide methemoglobin S

## Claims

1. A method for analyzing hemoglobin S by cation-exchange high-performance liquid chromatography,
the method being **characterized by** utilizing an eluent that contains an azide or a cyanide at a concentration of 0.1 to 50 mmol/L and has a pH of 6.80 to 7.50 for detecting one elution peak of eluted hemoglobin S in cation-exchange high-performance liquid chromatography.

2. The method for analyzing hemoglobin S by cation-according to claim 1,
wherein the eluent contains a salt at a concentration of 500 mmol/L or lower.

3. The method for analyzing hemoglobin S by cation-according to claim 1 or 2,
wherein the eluent contains a buffering agent at a concentration of 5 to 500 mmol/L.

## Patentansprüche

1. Verfahren zur Analyse von Hämoglobin S durch Kationenaustausch-Hochleistungsflüssigkeitschromatographie,
wobei das Verfahren durch die Verwendung eines Elutionsmittels gekennzeichnet ist, das ein Azid oder ein Cyanid in einer Konzentration von 0,1 bis 50 mmol/l enthält und einen pH-Wert von 6,80 bis 7,50 aufweist, zur Erfassung eines Elutionspeaks von eluiertem Hämoglobin S in der Kationenaustausch-Hochleistungsflüssigkeitschromatographie.

2. Verfahren zum Analysieren von Hämoglobin S durch Kationen nach Anspruch 1,
wobei das Elutionsmittel ein Salz in einer Konzentration von 500 mmol/l oder weniger enthält.

3. Verfahren zur Analyse von Hämoglobin S durch Kationen nach Anspruch 1 oder 2,
wobei das Elutionsmittel ein Puffermittel in einer Konzentration von 5 bis 500 mmol/l enthält.

## Revendications

1. Procédé pour l'analyse d'hémoglobine S par chromatographie liquide haute performance échangeuse de cations,
le procédé étant **caractérisé par** l'utilisation d'un éluant qui contient un azide ou un cyanure à une concentration de 0,1 à 50 mmol/l et présente un pH de 6,80 à 7,50 pour détecter un pic d'élution d'hémoglobine S éluée dans une chromatographie liquide haute performance échangeuse de cations.

2. Procédé pour l'analyse d'hémoglobine S par cations selon la revendication 1,
dans lequel l'éluant contient un sel à une concentration de 500 mmol/l ou inférieure.

3. Procédé pour l'analyse d'hémoglobine S par cations selon la revendication 1 ou 2,
dans lequel l'éluant contient un agent tampon à une concentration de 5 à 500 mmol/l.
